# EUROPEAN PATENT SPECIFICATION

(11) **EP 2 063 853 B1**
(45) Date of publication and mention of the grant of the patent: **21.02.2018**
(21) Application number: 07821282.6
(22) Date of filing: 12.10.2007
(51) Int. Cl.: A61K 8/23, A61K 8/26, A61K 8/31, A61K 8/34, A61K 8/35, A61K 8/42, A61K 8/49, A61K 8/96, A61Q 5/00, A61Q 13/00, A61K 8/92, A61K 8/97, A61K 8/36

(54) **USE OF C10-C14-ALKANEDIOLS IN THE PREPARATION OF A COMPOSITION FOR THE PROPHYLAXIS AND/OR TREATMENT OF MALASSEZIA-INDUCED DANDRUFF FORMATION AND COMPOSITIONS COMPRISING C10-C14-ALKANEDIOLS**
VERWENDUNG VON C10-C14-ALKANDIOLEN BEI DER HERSTELLUNG EINER ZUSAMMENSETZUNG ZUR PROPHYLAXE UND/ODER BEHANDLUNG VON SCHUPPENBILDUNG DURCH MALASSEZIA UND C10-C14-ALKANDIOLE ENTHALTENDE ZUSAMMENSETZUNGEN
UTILISATION DE C10-C14-ALCANEDIOLS POUR L'ÉLABORATION D'UNE COMPOSITION DESTINÉE À LA PROPHYLAXIE ET/OU AU TRAITEMENT DE L'APPARITION DE PELLICULES DÉCLENCHÉE PAR MALASSEZIA ET COMPOSITIONS À BASE DE C10-C14-ALCANEDIOLS

(30) Priority: 20.10.2006 US 852977 P; 20.10.2006 EP 06122667
(43) Date of publication of application: 03.06.2009
(73) Proprietor: Symrise AG, 37603 Holzminden (DE)
(72) Inventor: SCHMAUS, Gerhard, 37671 Höxter-Bosseborn (DE); LANGE, Sabine, 37603 Holzminden (DE)
(74) Representative: Fabry, Bernd
(86) International application number: PCT/EP2007/060915
(87) International publication number: WO 2008/046795

(56) References cited:
- EP-A- 1 598 064
- EP-A1- 0 524 548
- EP-A1- 1 269 992
- EP-A2- 0 584 692
- WO-A-97/30692
- WO-A-03/069994
- WO-A-2005/123028
- WO-A-2006/045743
- DE-A1- 10 341 179
- DE-A1- 19 927 891
- FR-A- 2 866 234
- FR-A1- 2 771 632
- FR-A1- 2 857 222
- JP-A- 2004 352 688
- US-A- 4 294 728
- WPI WORLD PATENT INFORMATION DERWENT, DERWENT, GB, vol. 39, no. 76, 11 August 1976 (1976-08-11), XP002022306 & JP 51 091327 A (KAO SOAP KK) 11 August 1976 (1976-08-11)
- WARNER K S ET AL: "Influences of Alkyl Group Chain Length and Polar Head Group on Chemical Skin Permeation Enhancement" JOURNAL OF PHARMACEUTICAL SCIENCES, AMERICAN PHARMACEUTICAL ASSOCIATION. WASHINGTON, US, vol. 90, no. 8, August 2001 (2001-08), pages 1143-1153, XP002428885 ISSN: 0022-3549
- DATABASE GNPD [Online] MINTEL 01 February 2005 BAIN DE TERRE: 'Thickening Mist' Database accession no. 10206594

## Description

The invention relates to the use of one, two, three or more C10-C14-alkane-1,2-diols, in particular 1,2-decanediol, 1,2-dodecanediol and/or 1,2-tetradecanediol, in the preparation of a composition for the prophylaxis and/or treatment of Malassezia-induced dandruff formation, as well as to cosmetic and/or dermatological preparations comprising one, two, three or more C10-C14-alkane-1,2-diols.

Dandruff is one of the most frequent skin problems, affecting more than 50 % of men and women worldwide. The formation of dandruff can be triggered *inter alia* by the following factors:
- by functional disturbances of the sebaceous glands caused by hereditary factors,
- by diseases of internal organs or a disturbance in their functioning,
- as a side-effect of medicaments,
- by stress, strain on the nerves and psychological problems,
- by drying out of the scalp by unsuitable care products, bacteria and fungi, and
- by increased sebum production by the scalp.

Dandruff in most cases forms as a result of overproduction of horny cells. This overproduction is triggered by tiny centres of inflammation of the scalp, which are visible only under a microscope. Because of the inflammation, the horny cells of the scalp do not mature fully, so that they are shed prematurely in large cell structures - as dandruff.

A frequent cause of dandruff formation is increased colonisation with bacteria or fungi. Malassezia species, such as, for example, *Malassezia furfur* or *Malassezia globosa,* are of particular relevance here. Malassezia species feed on saturated fatty acids, which they obtain by means of lipases that they release themselves, by breaking down the triglycerides that occur in human skin sebum. However, when the triglycerides are broken down, large amounts of unsaturated fatty acids are also released, which are not metabolised by the fungus and therefore accumulate in the sebum and consequently in some cases cause considerable irritation to the scalp. The increased growth of Malassezia species, such as, for example, *M. globosa* and *M*. *furfur,* can accordingly be regarded as one of the main causes of dandruff formation accompanied by considerable redness and considerable itching of the scalp. Redness of the scalp and itching can be encouraged still further by increased sebum production (seborrhoea).

When treating increased dandruff formation, attempts are made, for example, to remove the dandruff, to reduce the overproduction of horny cells and/or to inhibit the growth of fungi. There are used, *inter alia,* salicylic-acid-containing therapeutic agents, antimycotics having good activity against Malassezia species, tar-containing externals, sulfur and/or selenium disulfide.

Inhibiting the growth of fungi of the genus *Malassezia* is one of the main methods of controlling dandruff formation. An important group of active ingredients that are currently being used in the treatment of dandruff are antimycotics for topical and systemic administration from the group of the azoles. Preferred anti-dandruff agents are climbazole as well as further azoles such as, for example, benzimidazole, benzothiazole, bifonazole, butaconazole nitrate, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, thioconazole, as well as diazoles and triazoles, such as, for example, terconazole and itraconazole, as well as any desired combinations of the mentioned azoles. In particular because of the occurrence of sensitisation reactions, which is to be observed more frequently in clinical practice, but also because of the formation of potentially resistant strains, the antimycotics conventional in dandruff treatment are, however, increasingly becoming the focus of discussion.

Specific organic carboxylic acids, such as, for example, salicylic acid, are also used in anti-dandruff agents. Because the dosages used are in some cases very high, however, very considerable strain is put on the skin, and this manifests itself in particular as pronounced drying out of the skin, which can in some cases likewise be accompanied by considerable skin irritation owing to the pronounced reduction in the pH value of the skin.

Coal tar is used as a further anti-dandruff agent, but it has likewise come in for criticism because of cancerogenic side-effects.

The object of the present invention was, therefore, to provide a compound that is active against Malassezia species and at the same time is mild and tolerated by the skin, in particular is non-irritant, non-caustic, non-sensitising and/or non-cancerogenic and acts at a skin-friendly pH value.

The object is achieved at least partly by the subject-matters of the present invention, in particular as described in the present claims.

An embodiment of the present invention relates to the use of one, two, three or more C10-C14-alkane-1,2-diols in the preparation of a composition for the prophylaxis and/or treatment of Malassezia-induced dandruff formation.

A further embodiment of the present invention relates to a cosmetic and/or dermatological preparation for topical application, comprising or consisting of:
a) a dandruff-reducing amount of a total concentration of from 0.1 to 10 wt.% of one, two, three, four or more C10-C14-alkane-1,2-diols, in each case based on the total weight of the preparation, and
b) a dandruff-reducing amount of one, two, three, four or more anti-dandruff agents selected from the group consisting of climbazole, benzimidazole, benzothiazole, bifonazole, butaconazole nitrate, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, thioconazole, itraconazole, zinc pyrithione, coal tar, sulfur, selenium disulfide, aluminium chloride, octopirox, cyclopiroxolamine, undecylenic acid and the metal salts thereof, urea derivatives, griseofulvin, 8-hydroxyquinoline, ciloquinol, thiobendazole, thiocarbamates, triclosan, haloprogin, polyenes, hydroxypyridone, morpholine, benzylamine, allylamines, tea tree oil, clove leaf oil, coriander oil, palmarosa oil, thyme oil, oil of cinnamon as well as essential oil of bitter orange, cinnamaldehyde, citronellic acid, farnesol, berberine, hinokitiol, tropolone, birch tar oils, ichthyol, Sensiva SC-50 (ethylhexylglycerin), polyglycerol esters, arylalkyl alcohols, 3-phenyl-1-propanol, vetikol, muguet alcohol, Elestab HP-100 (hexamidine diisethionate), azelaic acid, , isothiazolinone and iodopropinylbutyl-carbamate, and
c) optionally one, two or more auxiliary substances and/or additives.

A further embodiment of the present invention relates to a fragrance composition comprising or consisting of:
a) a sensorially effective amount of a fragrance or of the sum of two, three, four or more fragrances,
b) a dandruff-reducing amount of one, two, three or more, preferably one, two or three, C10-C14-alkane-1,2-diols according to the invention, more preferably of a compound or mixture of two or three compounds selected from the group consisting of 1,2-decanediol, 1,2-dodecanediol and 1,2-tetradecanediol, and
c) optionally one or more carriers and/or additives.

Within the scope of the present invention, the C10-C14-alkane-1,2-diols contain an unbranched alkane chain having from 10 to 14 carbon atoms, the OH substituents being present in the 1- and 2-positions, and both (a) enantiomers having the 2S configuration and (b) enantiomers having the 2R configuration as well as (c) any desired mixtures of C10-C14-alkane-1,2-diols having the 2S and 2R configuration are included. Although it is particularly advantageous for commercial reasons to use racemates of C10-C14-alkane-1,2-diols for the prophylaxis and/or treatment of Malassezia-induced dandruff formation, because they are particularly easy to obtain by synthesis, the pure enantiomers or non-racemic mixtures of those enantiomers are likewise preferably suitable for the purposes according to the invention.

The improvement in the condition of skin having a tendency to Malassezia-induced dandruff is based, as has been shown by the study of the biological properties of alkane-1,2-diols (see Example 1), substantially on the property of the C10-C14-alkane-1,2-diols according to the invention of inhibiting the growth of fungi of the genus *Malassezia* (e.g. *Malassezia furfur,* synonym: *Pityrosporum ovale*), which are significantly involved in dandruff formation.

Preference is given to the use according to the invention of one or more, preferably one, two, three or more, C10-C14-alkane-1,2-diols, more preferably of a compound or a mixture of two or three compounds selected from the group consisting of 1,2-decanediol, 1,2-dodecanediol and 1,2-tetradecanediol.

The prior art regarding the antimicrobial action of aliphatic 1,2-diols in general, and in particular their possible uses in inhibiting the growth of representatives of the genus *Malassezia,* such as, for example, *Malassezia furfur* (synonym: *Pityrosporum ovale),* is described in greater detail below.

Although the antimicrobial action of aliphatic alkane-1,2-diols *per se* is already sufficiently well known, no action in inhibiting Malassezia species is described.

For example, particular references are known from JP 51 91327 to the bacteriostatic action of alkane-1,2-diols against particular microorganisms in connection with the preservation of foodstuffs and cosmetics, but no antimicrobial action of the alkane-1,2-diols against Malassezia species (e.g. *Malassezia furfur,* synonym: *Pityrosporum ovale*), the microorganisms that play a part in dandruff formation, is disclosed.

In addition, JP 2002/2003330 describes the antibacterial action of a combination of 1,2-alkanediols and ascorbic acid esters, but there is no disclosure in this specification of an antimicrobial action of the alkane-1,2-diols against Malassezia species (e.g. *Malassezia furfur,* synonym: *Pityrosporum ovale*), the microorganisms that play a part in dandruff formation.

From JP 11 322591 it is known only that specific 1,2-alkanediols having a chain length of from 4 to 10 carbon atoms can be used in order to reduce the dose of conventional antiseptic microbicides in an antimicrobial formulation. 1,2-Pentanediol and 1,2-hexanediol as well as 1,2-octanediol are mentioned as being preferred. However, the Japanese specification contains no reference to the action of alkane-1,2-diols against Malassezia species (e.g. *Malassezia furfur,* synonym: *Pityrosporum ovale),* which are significantly involved in dandruff formation.

FR 2,771,632 discloses that specific alkane-1,2-diols having a chain length of from 8 to 18 carbon atoms can be used in admixture with an N-acylamino acid as a composition against acne and dandruff formation as well as for the disinfection of skin and mucosa. Surprisingly, 1,2-octanediol is mentioned as being preferred.

US 4,294,728 describes 1,2-diols as agents for improving foaming characteristics, whereby better hair detangling is achieved. However, this specification contains no reference to the action of alkane-1,2-diols against Malassezia species (e.g. *Malassezia furfur,* synonym: *Pityrosporum ovale),* which are significantly involved in dandruff formation.

EP 0 584 692 relates to agents for cleansing and conditioning the hair, the skin, etc., which agents contain alkane-1,2-diols having a chain length of from C10 to C16. The agents described therein exhibit improved foaming properties and an improved sensation on the skin and are likewise outstandingly suitable as moisturising agents (conditioning agents). However, there is no reference in this specification to the action of alkane-1,2-diols against Malassezia species (e.g. *Malassezia furfur,* synonym: *Pityrosporum ovale*), which are significantly involved in dandruff formation.

WO 00/00164 describes only alkane-1,2-diols having a chain length of from C5 to C8 for reducing frizzy hair (smoothing the hair). There is likewise no reference in this specification to the action of alkane-1,2-diols against Malassezia species (e.g. *Malassezia furfur,* synonym: *Pityrosporum ovale*), which are significantly involved in dandruff formation.

WO 03/000220 (Dragoco Gerberding GmbH & Co. KG) describes the use of 1,2-decanediol solely against bacteria that cause body odour. This specification contains no reference to the action of alkane-1,2-diols against Malassezia species (e.g. *Malassezia furfur,* synonym: *Pityrosporum ovale*), which are significantly involved in dandruff formation.

EP 0 524 548 discloses specific antimicrobially active mixtures which, as well as containing (A) an antimicrobially active aromatic alcohol of formula 1 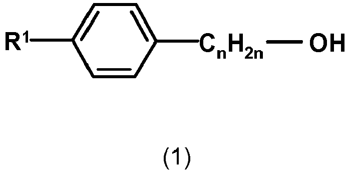 in which R¹ is hydrogen or an alkyl group having from 1 to 4 carbon atoms and n is an integer from 1 to 6, contain (B) an antimicrobially active 1,2- or 1,3-diol of the formula R²-CHOH-(CHR³)ₓ-CH₂OH, wherein x = 0 or 1 and when x = 0, R² is an alkyl group having from 6 to 22 carbon atoms or an alkoxymethyl or 2-hydroxyalkoxymethyl group having in each case from 6 to 22 carbon atoms in the alkoxy group, and when x = 1, the group R² is hydrogen, and R³ has one of the above-mentioned meanings of R², the components being present in a ratio by weight of from 9:1 to 1:9. The disclosed antimicrobially active mixtures are described as being suitable for the preparation of antiseptically active skin cleansing agents and for the preservation of aqueous preparations of microbially degradable or perishable substances. There is no reference in EP 0 524 548 to an action of alkane-1,2-diols against specific fungi that are involved in dandruff formation, such as especially Malassezia species (e.g. *Malassezia furfur,* synonym: *Pityrosporum ovale*).

In US 6,123,953 it is stated that unbranched 1,2-alkanediols having a total chain length of from 5 to 14 carbon atoms and preferably from 6 to 8 carbon atoms are suitable for topical application to the skin and in particular for treating acne, for treating capillary layers, for disinfecting small wounds (scratches), for disinfecting the hands of surgeons and patients and for disinfecting the udders of milk-yielding animals. US 6,123,953 further discloses that formulations which, as well as containing an alkanediol, contain a gel of the glyceryl polymethacrylate type, can be used in a low concentration for treating acne, skin problems, impetigo, microorganism-based body odours, athlete's foot and the like. This usability is attributed to a synergistic interaction between the gel and the alkanediol. However, 1,2-octanediol is named in this specification as the preferred alkanediol for treating acne. US 6,123,953 does not contain any reference to an action of alkane-1,2-diols, especially alkane-1,2-diols having a chain length of from C10 to C14, against specific fungi that are involved in dandruff formation, such as especially *Malassezia furfur* (synonym: *Pityrosporum ovale*). WO03/069994 discloses the use of 1,2-alkanediol (C5-C10) mixtures as antimicrobial agent e.g. for treating body odor, acne or mycoses or for preserving perishable products. EP 1 598 064 discloses the use of 1,2-decanediol in combination with alpha-hydroxy acids in anti-acne products. EP 1 598 064 contains no reference to an action of alkane-1,2-diols against specific fungi that are involved in dandruff formation, such as especially *Malassezia furfur* (synonym: *Pityrosporum ovale*). According to the present invention it is possible to select one, two, three or more, preferably one, two or three, C10-C14-alkane-1,2-diols, more preferably a compound or a mixture of two or three compounds from the group consisting of 1,2-decanediol, 1,2-dodecanediol and 1,2-tetradecanediol. The preferred alternatives of the C10-C14-alkane-1,2-diols are to be applied to all subject-matters of the present invention.

Preference is likewise given to a preparation according to the invention, characterised in that one, two or more auxiliary substances and/or additives are selected from the group of the fragrances. It has been found to be particularly advantageous that the C10-C14-alkane-1,2-diols according to the invention, in particular 1,2-decanediol, 1,2-dodecanediol and/or 1,2-tetradecanediol, each possess only a weak inherent odour or are even odourless and are therefore predisposed not to interfere with the use of the fragrance composition according to the invention.

The C10-C14-alkane-1,2-diols according to the invention are used particularly advantageously in cosmetic and/or dermatological preparations. It is advantageous if the content of one, two, three or more, preferably one, two or three, C10-C14-alkane-1,2-diols according to the invention in the cosmetic and/or dermatological preparations are used in a total concentration of from 0.1 to 10 wt.%, preferably in a concentration of from 0.2 to 5 wt.% and very particularly preferably in a concentration of from 0.5 to 4 wt.%, in each case based on the total weight of the preparation.

One, two or more anti-dandruff agents are preferably used in a preparation according to the invention in addition to one or more, preferably one, two or three, C10-C14-alkane-1,2-diols, more preferably in addition to a compound or a mixture of two or three compounds from the group consisting of 1,2-decanediol, 1,2-dodecanediol and 1,2-tetradecanediol.

One, two or more further anti-dandruff agents are preferably selected from the group of the azoles consisting of climbazole, benzimidazole, benzothiazole, bifonazole, butaconazole nitrate, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, thioconazole, as well as diazoles and triazoles, preferably terconazole and itraconazole, and any desired combinations of the mentioned azoles.

Alternatively or in addition to the azoles, pyrithione salts, preferably 1-hydroxy-2-pyrithione salts, can preferably also be used as anti-dandruff agents in combination with one or more C10-C14-alkane-1,2-diols according to the invention for the treatment of dandruff formation. Preference is given to pyrithione salts of the metal cations of sodium, zinc, tin, cadmium, magnesium, aluminium and zirconium. Particular preference is given to the zinc salt of 1-hydroxy-2-pyrithione (known as "zinc pyrithione" or "ZPT").

Alternatively or in addition to the azoles and/or pyrithione salts, one or more anti-dandruff agents can preferably be selected from the group consisting of coal tar; sulfur; selenium disulfide; aluminium chloride; octopirox (INCI: piroctone olamine); cyclopiroxolamine; undecylenic acid and the metal salts thereof; potassium permanganate; sodium thiosulfate, urea preparations, griseofulvin, 8-hydroxyquinoline, ciloquinol, thiobendazole; thiocarbamates; triclosan; haloprogin; polyenes; hydroxypyridone; morpholine; benzylamine; allylamines, preferably terbinafine; tea tree oil; clove leaf oil; coriander oil; palmarosa oil; thyme oil; oil of cinnamon as well as essential oil of bitter orange; cinnamaldehyde; citronellic acid; farnesol; berberine; hinokitiol; tropolone; birch tar oils; ichthyol (sulfonated shale oil), Sensiva SC-50 (ethylhexylglycerin); polyglycerol esters, preferably polyglycerol-3-caprylate; arylalkyl alcohols, preferably phenylethyl alcohol; 3-phenyl-1-propanol; vetikol (4-methyl-4-phenyl-2-pentanol); muguet alcohol 2,2-dimethyl-3-phenylpropanol; Elestab HP-100 (hexamidine diisethionate); azelaic acid; isothiazolinones, preferably octylisothiazolinone and iodopropinylbutyl-carbamate.

The person skilled in the art can supplement the above list with a large number of further anti-dandruff agents; the listed anti-dandruff agents can also be used in combination with one another. Preference is given to the use of one, two, three or more additional anti-dandruff agents in combination with one or more, preferably one, two or three, C10-C14-alkanediols according to the invention, more preferably with a compound or a mixture of two or three compounds from the group consisting of 1,2-decanediol, 1,2-dodecanediol and 1,2-tetradecanediol, in preparations for the treatment of dandruff formation, wherein the additional anti-dandruff agent(s) is/are selected from the group consisting of: climbazole, ketoconazole, zinc pyrithione, sulfur, selenium disulfide, octopirox (INCI: piroctone olamine), cyclopiroxolamine, undecylenic acid and the metal salts thereof, ichthyol (sulfonated shale oil), Sensiva SC-50 (ethylhexylglycerin) and/or vetikol (4-methyl-4-phenyl-2-pentanol).

Because dandruff formation is mostly accompanied by inflammatory processes, which manifest themselves especially in pronounced redness and itching of the scalp, cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can particularly advantageously also contain one, two or more anti-inflammatory and/or redness- and/or itching-alleviating active ingredients (anti-inflammatories). Any anti-inflammatory or redness- and/or itching-alleviating active ingredients suitable or conventional for cosmetic and/or dermatological applications can be used.

As anti-inflammatory or redness- and/or itching-alleviating active ingredients there are advantageously used one, two, three or more steroidal anti-inflammatory active ingredients of the corticosteroid type, preferably hydrocortisone, hydrocortisone derivatives such as hydrocortisone 17-butyrate, dexamethasone, dexamethasone phosphate, methylprednisolone or cortisone, it being possible for this list to be extended by adding further steroidal anti-inflammatories.

Additionally, the C10-C14-alkanediols according to the present invention themselves, preferably a compound or a mixture of two or three compounds from the group consisting of 1,2-decanediol, 1,2-dodecanediol and 1,2-tetradecanediol, in particular 1,2-decanediol or a mixture of two or three compounds comprising1,2-decanediol, have redness- and/or itching-alleviating activity. This positive property is particularily observed when said C10-C14-alkanediols are incorporated into leave-on compositions for the prophylaxis and/or treatment of *Malassezia* -induced dandruff information.

Alternatively or in addition, it is possible to use one, two, three or more non-steroidal anti-inflammatories selected from the group consisting of oxicams, preferably piroxicam or tenoxicam; salicylates, preferably aspirin, disalcid, solprin or fendosal; acetic acid derivatives, preferably diclofenac, fenclofenac, indomethacin, sulindac, tolmetin or clindanac; fenamates, preferably mefenamic, meclofenamic, flufenamic or niflumic; propionic acid derivatives, preferably ibuprofen, naproxen, benoxaprofen, or pyrazoles, such as phenylbutazone, oxyphenylbutazone, febrazone or azapropazone.

Alternatively or in addition, naturally occurring anti-inflammatory or redness- and/or itching-alleviating active ingredients can be used. It is possible to use plant extracts, special highly active plant extract fractions, as well as highly pure active substances isolated from plant extracts. Particular preference is given to extracts, fractions and active substances from camomile, aloe vera, Commiphora species, Rubia species, willow, willow-herb, oats, Calendula, arnica, St. John's wort, honeysuckle, rosemary, Melissa, ginger, Passiflora incarnata, witch hazel, Pueraria, Dianthus or Echinacea, as well as pure substances such as, *inter alia,* bisabolol, apigenin, apigenin-7-glucoside, rosemary acid, boswellic acid, phytosterols, glycyrrhizinic acid, glabridin, licochalcone A, gingerols and anthranilic acid amides, particularly preferably avenanthramide or dianthramide.

The amount of anti-inflammatory or redness- and/or itching-alleviating active ingredients (one or more compounds) in the preparations is preferably from 0.0001 to 20 wt.%, particularly preferably from 0.0001 to 10 wt.%, especially from 0.001 to 5 wt.%, in each case based on the total weight of the preparation.

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can be used in cosmetic and/or dermatological formulations having very different forms. For example, they can be a solution (e.g. aqueous, aqueous-alcoholic or alcoholic solution), an emulsion of the water-in-oil (W/O) or oil-in-water (O/W) type or a multiple emulsion, for example of the water-in-oil-in-water (W/O/W) or oil-in-water-in-oil (O/W/O) type (in each case also in the form of silicone emulsions), a hydrodispersion or lipodispersion, a Pickering emulsion, a solid stick or an aerosol. It can also be advantageous to impregnate a water-insoluble substrate (e.g. a cloth, a nonwoven, a pad) with C10-C14-alkane-1,2-diols according to the invention, it being possible for this form of application to be both a substrate having a dry appearance and a substrate having a moist appearance. Further advantageous forms of administration of the C10-C14-alkane-1,2-diols according to the invention are creams, ointments, hydrodispersions, lotions, tinctures, pump sprays, aerosol sprays, aqueous solutions, cleansing substrates and the like. As well as containing water, the aqueous phase can also contain other ingredients, for example alcohols, diols or polyols having a low C number, and ethers thereof, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, also alcohols having a low C number, for example ethanol, isopropanol, 1,2-propanediol, glycerol, as well as, in particular, one or more thickening agents, which can advantageously be selected from the group silicon dioxide, aluminium silicates, polysaccharides and derivatives thereof, for example hyaluronic acid, xanthan gum, hydroxypropylmethylcellulose, particularly advantageously from the group of the polyacrylates, preferably a polyacrylate from the group of the so-called carbopols, for example carbopols of types 980, 981, 1382, 2984, 5984, in each case individually or in combination.

The C10-C14-alkane-1,2-diols according to the invention can be combined with a large number of further auxiliary substances and/or additives, resulting in preferred cosmetic and/or pharmaceutical mixtures or products.

### Combination with anti-acne products:

Cosmetic and/or dermatological preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can additionally contain one, two or more conventional anti-acne active ingredients, preferably selected from the group consisting of benzoyl peroxide, azelaic acid, salicylic acid, retinoids such as all-trans-retinic acid (tretinoin), all-trans-retinal or cis-13-retinic acid (isotretinoin), as well as antibiotics which are used specifically for the treatment of acne, such as, for example, clindamycin, erythromycin, tetracycline, doxycycline and/or sulfur. Chlorhexidine, triclosan, bisabolol, farnesol and phenoxyethanol as well as isoflavonoids (e.g. genistein, daidzein, genistin and glycitein) can also advantageously be used together with one or more, preferably one, two or three, C10-C14-alkane-1,2-diols according to the invention in the prophylaxis and/or treatment of Malassezia-induced dandruff formation.

### Combination with products that regulate skin moisture:

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can particularly advantageously additionally be combined with one, two, three or more compounds that regulate skin moisture. Cosmetic preparations containing C10-C14-alkane-1,2-diols according to the invention can therefore advantageously additionally contain one, two, three or more humectants selected from the group consisting of: sodium lactate; urea; urea derivatives; alcohols, preferably glycerol, further diols such as propylene glycol, hexylene glycol, 1,2-pentanediol or 1,2-hexanediol; collagen; elastin or hyaluronic acid; diacyl adipate; petrolatum; urocanic acid; lecithin; panthenol; phytantriol; lycopene; (pseudo)ceramides; glycosphingolipids; cholesterol; phytosterols; chitosan; chondroitin sulfate; lanolin; lanolin esters; amino acids; alpha-hydroxy acids (e.g. citric acid, lactic acid, malic acid) and derivatives thereof; mono-, di- and oligo-saccharides, preferably glucose, galactose, fructose, mannose, fruit sugars and lactose; polysugars, preferably β-glucans, in particular 1,3-1,4-β-glucan from oats; alpha-hydroxy fatty acids; triterpenic acids, preferably betulic acid or ursolic acid; algae extracts.

Depending on the substance, the use concentration of the humectants is in the concentration range from 0.1 to 10 wt.% and preferably in the concentration range from 0.5 to 5 wt.%, in each case based on the total weight of a ready-to-use cosmetic or pharmaceutical end product.

### Combination with cooling active ingredients:

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can particularly advantageously additionally be combined with one, two, three or more cooling active ingredients. Individual cooling active ingredients that are preferably used within the scope of the present invention are listed hereinbelow. The person skilled in the art can supplement the following list with a large number of further cooling active ingredients; the listed cooling active ingredients can also be used in combination with one another: I-menthol, d-menthol, racemic menthol, menthone glycerol acetal (trade name: Frescolat®MGA), menthyl lactate (trade name: Frescolat®ML; menthyl lactate is preferably I-menthyl lactate, in particular I-menthyl I-lactate), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxyethylmenthyl carbonate, 2-hydroxypropylmenthyl carbonate, N-acetylglycine menthyl ester, isopulegol, menthylhydroxycarboxylic acid esters (e.g. menthyl 3-hydroxy butyrate), monomenthyl succinate, 2-mercaptocyclodecanone, menthyl-2-pyrrolidin-5-one carboxylate, 2,3-dihydroxy-p-menthane, 3,3,5-trimethylcyclohexanone glycerol ketal, 3-menthyl 3,6-di- and -tri-oxaalkanoates, 3-menthyl methoxyacetate, icilin.

Preferred cooling active ingredients are: I-menthol, d-menthol, racemic menthol, menthone glycerol acetal (trade name: Frescolat®MGA), menthyl lactate (preferably I-menthyl lactate, in particular I-menthyl I-lactate, trade name: Frescolat®ML), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxyethylmenthyl carbonate, 2-hydroxypropylmenthyl carbonate, isopulegol.

Particularly preferred cooling active ingredients are: I-menthol, racemic menthol, menthone glycerol acetal (trade name: Frescolat®MGA), menthyl lactate (preferably I-menthyl lactate, in particular I-menthyl I-lactate, trade name: Frescolat®ML), 3-menthoxypropane-1,2-diol, 2-hydroxyethylmenthyl carbonate, 2-hydroxypropylmenthyl carbonate.

Most particularly preferred cooling active ingredients are: I-menthol, menthone glycerol acetal (trade name: Frescolat®MGA), menthyl lactate (preferably I-menthyl lactate, in particular I-menthyl I-lactate, trade name: Frescolat®ML).

Depending on the substance, the use concentration of the cooling active ingredients that are to be used is preferably in the concentration range from 0.01 to 20 wt.% and more preferably in the concentration range from 0.1 to 5 wt.%, based on the total weight of a ready-to-use cosmetic or pharmaceutical end product.

### Combination with osmolytes:

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also be used together with osmolytes. Examples of osmolytes which may be mentioned include: substances from the group of the sugar alcohols (myo-inositol, mannitol, sorbitol), quaternary amines such as taurine, choline, betaine, betaine-glycine, ectoin, diglycerol phosphate, phosphorylcholine, glycerophosphorylcholine, amino acids such as glutamine, glycine, alanine, glutamate, aspartate or proline, phosphatidylcholine, phosphatidylinositol, inorganic phosphates, and also polymers of the mentioned compounds, such as proteins, peptides, polyamino acids and polyols. All osmolytes at the same time have a skin-moisturising action.

### Combination with caring substances:

Caring substances which can be combined with one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, include animal and/or vegetable fats and oils such as olive oil, sunflower oil, refined soya oil, palm oil, sesame oil, rapeseed oil, almond oil, borage oil, evening primrose oil, coconut oil, shea butter, jojoba oil, sperm oil, beef tallow, neat's foot oil and lard, as well as optionally further caring constituents such as, for example, fatty alcohols having from 8 to 30 carbon atoms. The fatty alcohols used can be saturated or unsaturated and linear or branched.

Caring substances which can particularly preferably be combined with the C10-C14-alkane-1,2-diols according to the invention additionally include in particular also
- ceramides, ceramides being understood as being N-acylsphingosines (fatty acid amides of sphingosine) or synthetic analogues of such lipids (so-called pseudoceramides), which markedly improve the water-retaining capacity of the stratum corneum
- phospholipids, for example soya lecithin, egg lecithin and cephalins
- vaseline, paraffin and silicone oils; the latter include *inter alia* dialkyl- and alkylaryl-siloxanes such as dimethylpolysiloxane and methylphenylpolysiloxane, as well as alkoxylated and quaternised derivatives thereof.

### Combination with preservatives or chelators:

Cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also contain one, two, three or more active ingredients for preserving cosmetic products as well as perspiration-inhibiting active ingredients (antiperspirants) and (metal) chelators.

Preference is given to one, two, three or more preservatives selected from the group consisting of benzoic acid and its esters and salts, propionic acid and its esters and salts; salicylic acid and its esters and salts, 2,4-hexadienoic acid (sorbic acid) and its esters and salts; formaldehyde and paraformaldehyde, 2-hydroxy biphenyl ether and its salts, 2-zinc sulfidopyridine N-oxide, inorganic sulfites and bisulfites, sodium iodate, chlorobutanol, 4-ethylmercury-(II)5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy diphenyl ether, 4-chloro-3,5-dimethylphenol, 1,1'-methylene-bis(3-(1-hydroxymethyl-2,4-dioximidazolidin-5-yl)urea), poly-(hexa-methylenediguanide) hydrochloride, 2-phenoxyethanol, hexamethylenetetramine, 1-(3-chloroallyl)-3,5,7-triaza-1-azoniaadamantane chloride, 1(4-chloro-phenoxy)1(1H-imidazol-1-yl)-3,3-dimethyl-2-butanone, 1,3-bis-(hydroxy-methyl)-5,5-dimethyl-2,4-imidazolidinedione, benzyl alcohol, octopirox, 1,2-dibromo-2,4-dicyanobutane, 2,2'-methylene-bis(6-bromo-4-chlorophenol), bromochlorophene, mixture of 5-chloro-2-methyl-3(2H)-isothiazolinone and 2-methyl-3(2H)-isothiazolinone with magnesium chloride and magnesium nitrate, 2-benzyl-4-chlorophenol, 2-chloroacetamide, chlorhexidine chlorhexidine acetate, chlorhexidine gluconate, chlorhexidine hydrochloride, 1-phenoxy-propan-2-ol, N-alkyl(C₁₂-C₂₂)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chloro-phenoxy)-1,2-propanediol, hyamine, alkyl-(C₈-C₁₈)-dimethyl-benzyl-ammonium chloride, alkyl-(C₈-C₁₈)-dimethyl-benzylammonium bromide, alkyl-(C₈-C₁₈)-dimethyl-benzyl-ammonium saccharinate, benzylhemiformal, 3-iodo-2-propinyl-butyl carbamate or sodium hydroxymethyl-aminoacetate.

Preferred (metal) chelators to be used are *inter alia* α-hydroxy fatty acids, phytic acid, lactoferrin, α-hydroxy acids and their salts, preferably citric acid or malic acid, as well as galactaric acid, galacturonic acid, gluconic acid, glucuronic acid, ribonic acid and its salts, humic acids, bile acids, bile extracts, bilirubin, biliverdin or EDTA, EGTA and derivatives thereof.

### Combination with animal and/or vegetable protein hydrolysates:

Animal and/or plant protein hydrolysates can advantageously also be added to one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol. Particularly advantageous animal and/or plant protein hydrolysates are elastin, collagen, keratin, milk protein, soya protein, oat protein, pea protein, almond protein and wheat protein fractions or corresponding protein hydrolysates, as well as the condensation products thereof with fatty acids, and also quaternised protein hydrolysates, the use of plant protein hydrolysates being preferred.

### Combination with solvents:

If a cosmetic and/or dermatological preparation containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, is a solution or lotion, the following can be used as solvents:
- water or aqueous solutions;
- fatty oils, fats, waxes and other natural and synthetic fatty bodies, preferably esters of fatty acids with alcohols having a low C number, e.g. with isopropanol, propylene glycol or glycerol, or esters of fatty alcohols with alkanoic acids having a low C number or with fatty acids;
- alcohols, diols or polyols having a low C number, and their ethers, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products.

In particular, mixtures of the above-mentioned solvents are used. In the case of alcoholic solvents, water can be a further constituent.

### Combination with antioxidants:

Cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also contain one, two, three or more antioxidants, it being possible to use any antioxidants suitable or conventional for cosmetic and/or dermatological applications.

### Combination with vitamins:

Cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also contain one, two, three or more vitamins and/or vitamin precursors, it being possible to use any vitamins or vitamin precursors suitable or conventional for cosmetic and/or dermatological applications.

### Combination with skin-lightening agents:

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can in many cases advantageously be used in combination with one, two, three or more skin-lightening active ingredients. According to the invention, it is possible to use any skin-lightening active ingredients suitable or conventional for cosmetic and/or dermatological applications. Advantageous skin-lightening active ingredients are kojic acid (5-hydroxy-2-hydroxymethyl-4-pyranone), kojic acid derivatives such as, for example, kojic acid dipalmitate, arbutin, ascorbic acid, ascorbic acid derivatives, hydroquinone, hydroquinone derivatives, resorcinol, sulfur-containing molecules such as, for example, glutathione or cysteine, alpha-hydroxy acids (e.g. citric acid, lactic acid, malic acid) and derivatives thereof, N-acetyl-tyrosine and derivatives thereof, undecenoylphenylalanine, gluconic acid, 4-alkylresorcinols, diphenylmethane derivatives such as, for example, 4-(1-phenylethyl)1,3-benzenediol, chromone derivatives such as aloesin, flavonoids, thymol derivatives, 1-aminoethylphosphinic acid, thiourea derivatives, ellagic acid, nicotinamide, zinc salts such as, for example, zinc chloride or zinc gluconate, thujaplicin and its derivatives, triterpenes such as maslinic acid, sterols such as ergosterol, benzofuranones such as senkyunolide, vinyl- and ethyl-guiacol, inhibitors of nitrogen oxide synthesis such as, for example, L-nitroarginine and its derivatives, 2,7-dinitroindazole or thiocitrulline, metal chelators (e.g. α-hydroxy fatty acids, palmitic acid, phytinic acid, lactoferrin, humic acid, bile acid, bile extracts, bilirubin, biliverdin, EDTA, EGTA and derivatives thereof), retinoids, soya milk, serine protease inhibitors or liponic acid or other synthetic or natural active ingredients for lightening the skin and hair, it being possible for the latter also to be used in the form of an extract from plants, such as, for example, bearberry extract, rice extract, liquorice root extract or constituents concentrated therefrom, such as glabridine or licochalcone A, Artocarpus extract, extract of Rumex and Ramulus species, extracts from pine species (Pinus) and extracts from Vitis species or concentrated stilbene derivatives therefrom, extracts from saxifrage, mulberry, scutelleria and/or grape.

### Combination with skin-tanning agents:

Cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also contain one, two, three or more active ingredients having skin-tanning action. It is possible to use any skin-tanning active ingredients suitable or conventional for cosmetic and/or dermatological applications. Examples which may be mentioned here include dihydroxyacetone (DHA; 1,3-dihydroxy-2-propanone). DHA can be present in both monomeric and dimeric form, the proportion of dimers being predominant in crystalline form.

### Combination with saccharides:

Cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also contain one, two, three or more mono-, di- and oligo-saccharides, preferably glucose, galactose, fructose, mannose, fruit sugars and lactose.

### Combination with plant extracts:

Cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also contain one, two, three or more plant extracts, which are conventionally prepared by extraction of the whole plant but in some cases also solely from blossoms and/or leaves, wood, bark or roots of the plant.

### Combination with surfactants:

Cosmetic preparations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can, in particular when crystalline or microcrystalline solids, for example inorganic micropigments, are to be incorporated into the preparations, also contain one, two, three or more anionic, cationic, non-ionic and/or amphoteric surfactants. Surfactants are amphiphilic substances which are able to dissolve organic, non-polar substances in water. The hydrophilic components of a surfactant molecule are mostly polar functional groups, for example -COO⁻, -OSO₃²⁻, -SO₃⁻, while the hydrophobic portions are generally non-polar hydrocarbon radicals. Surfactants are generally classified according to the type and charge of the hydrophilic molecule portion. A distinction can be made between four groups:
- anionic surfactants,
- cationic surfactants,
- amphoteric surfactants and
- non-ionic surfactants.

Anionic surfactants generally contain carboxylate, sulfate or sulfonate groups as functional groups. In aqueous solution they form negatively charged organic ions in an acidic or neutral medium. Cationic surfactants are almost exclusively characterised by the presence of a quaternary ammonium group. In aqueous solution they form positively charged organic ions in an acidic or neutral medium. Amphoteric surfactants contain both anionic and cationic groups and accordingly behave in aqueous solution like anionic or cationic surfactants, depending on the pH value. In a strongly acidic medium they have a positive charge, and in an alkaline medium thay have a negative charge. In the neutral pH range, on the other hand, they are zwitterionic. Typical of non-ionic surfactants are polyether chains. Non-ionic surfactants do not form ions in an aqueous medium.

### A. Anionic surfactants

Anionic surfactants which are advantageously to be used are acylamino acids (and their salts), such as
- acyl glutamates, for example sodium acyl glutamate, di-TEA palmitoyl aspartate and sodium caprylic/capric glutamate,
- acyl peptides, for example palmitoyl-hydrolysed milk protein, sodium cocoyl-hydrolysed soya protein and sodium/potassium cocoyl-hydrolysed collagen,
- sarcosinates, for example myristoyl sarcosine, TEA-lauroyl sarcosinate, sodium lauroyl sarcosinate and sodium cocoyl sarcosinate,
- taurates, for example sodium lauroyl taurate and sodium methylcocoyl taurate,
- acyl lactylates, lauroyl lactylate, caproyl lactylate, stearoyl lactylate,
- alaninates,
carboxylic acids and their derivatives, such as
for example lauric acid, aluminium stearate, magnesium alkanolate and zinc undecylenate,
- ester carboxylic acids, for example calcium stearoyl lactylate, laureth-6 citrate and sodium PEG-4 lauramide carboxylate,
- ether carboxylic acids, for example sodium laureth-13 carboxylate and sodium PEG-6 cocamide carboxylate,
phosphoric acid esters and their salts, such as, for example, DEA-oleth-10 phosphate and dilaureth-4 phosphate,
sulfonic acids and their salts, such as
- acyl isothionates, e.g. sodium/ammonium cocoyl isethionate,
- alkylarylsulfonates,
- alkylsulfonates, for example sodium cocomonoglyceride sulfate, sodium C₁₂₋₁₄ olefin sulfonate, sodium lauryl sulfoacetate and magnesium PEG-3 cocamide sulfate,
- sulfosuccinates, for example dioctyl sodium sulfosuccinate, disodium laureth sulfosuccinate, disodium lauryl sulfosuccinate and disodium undecyleneamido-MEA sulfosuccinate
and also
sulfuric acid esters, such as
- alkyl ether sulfate, for example sodium, ammonium, magnesium, MIPA, TIPA laureth sulfate, sodium myreth sulfate and sodium C12-13 pareth sulfate,
- alkyl sulfates, for example sodium, ammonium and TEA lauryl sulfate.

### B. Cationic surfactants

Cationic surfactants which are advantageously to be used are
- alkylamines,
- alkylimidazoles,
- ethoxylated amines and
- quaternary surfactants.

   RNH₂CH₂CH₂COO⁻ (at pH=7)

   RNHCH₂CH₂COO- B⁺ (at pH=12) B⁺ = any cation, e.g. Na⁺
- ester quats

Quaternary surfactants contain at least one N atom covalently bonded to 4 alkyl or aryl groups. Regardless of the pH value, this results in a positive charge. Alkylbetaine, alkylamidopropylbetaine and alkylamidopropylhydroxysulfain are advantageous. The cationic surfactants used can further preferably be selected from the group of the quaternary ammonium compounds, in particular benzyltrialkylammonium chlorides or bromides, such as, for example, benzyldimethylstearylammonium chloride, also alkyltrialkylammonium salts, for example cetyltrimethylammonium chloride or bromide, alkyldimethylhydroxy-ethylammonium chlorides or bromides, dialkyldimethylammonium chlorides or bromides, alkylamide ethyltrimethylammonium ether sulfates, alkylpyridinium salts, for example lauryl- or cetyl-pyridinium chloride, imidazoline derivatives and compounds of cationic nature such as amine oxides, for example alkyldimethylamine oxides or alkylaminoethyldimethylamine oxides. Cetyltrimethylammonium salts are particularly advantageously to be used.

### C. Amphoteric surfactants

Amphoteric surfactants which are advantageously to be used are
- acyl-/dialkylethylenediamine, for example sodium acyl amphoacetate, disodium acyl amphodipropionate, disodium alkyl amphodiacetate, sodium acyl amphohydroxypropylsulfonate, disodium acyl amphodiacetate and sodium acyl amphopropionate,
- N-alkylamino acids, for example aminopropylalkylglutamide, alkyl-aminopropionic acid, sodium alkylimidodipropionate and lauroamphocar-boxyglycinate.

### D. Non-ionic surfactants

Non-ionic surfactants which are advantageously to be used are
- alcohols,
- alkanolamides, such as cocamides MEA/ DEA/ MIPA,
- amine oxides, such as cocoamidopropylamine oxide,
- esters formed by esterification of carboxylic acids with ethylene oxide, glycerol, sorbitan or other alcohols,
- ethers, for example ethoxylated/propoxylated alcohols, ethoxylated/propoxylated esters, ethoxylated/propoxylated glycerol esters, ethoxylated/propoxylated cholesterols, ethoxylated/propoxylated triglyceride esters, ethoxylated propoxylated lanolin, ethoxylated/propoxylated polysiloxanes, propoxylated POE ethers and alkylpolyglycosides such as lauryl glucoside, decyl glycoside and cocoglycoside,
- sucrose esters, sucrose ethers,
- polyglycerol esters, diglycerol esters, monoglycerol esters,
- methylglucose esters, esters of hydroxy acids.

It is also advantageous to use a combination of anionic and/or amphoteric surfactants with one or more non-ionic surfactants.

The surface-active substance can be present in the preparations according to the invention containing alkane-1,2-diols having a chain length of from C10 to C14 in a concentration of from 1 to 98 wt.%, based on the total weight of the preparations.

Emulsions comprising a mixture according to the invention:
Cosmetic or dermatological preparations containing alkane-1,2-diols having a chain length of from C10 to C14 according to the invention can also be present in the form of emulsions.

The oil phase can advantageously be selected from the following group of substances:
- mineral oils, mineral waxes;
- fatty oils, fats, waxes and other natural and synthetic solid bodies, preferably esters of fatty acids with alcohols having a low C number, e.g. with isopropanol, propylene glycol or glycerol, or esters of fatty alcohols with alkanoic acids having a low C number or with fatty acids;
- alkyl benzoates;
- silicone oils such as dimethylpolysiloxanes, diethylpolysiloxanes, diphenylpolysiloxanes and mixed forms thereof.

There can advantageously be used non-comedogenic active esters of saturated and/or unsaturated, branched and/or unbranched alkanecarboxylic acids having a chain length of from 3 to 30 carbon atoms and saturated and/or unsaturated, branched and/or unbranched alcohols having a chain length of from 3 to 30 carbon atoms. Preferred ester oils are 3,5,5-trimethylhexyl-3,5,5-trimethyl hexanoate, 2-ethylhexyl isononanoate, 2-ethylhexyl-3,5,5-trimethyl hexanoate, 2-ethylhexyl-2-ethyl hexanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate and also synthetic, semi-synthetic and natural mixtures of such esters, for example jojoba oil advantageously be combined with the alkane-1,2-diols having a chain length of from C10 to C14 according to the invention.

In addition, however, esters of aromatic carboxylic acids and saturated and/or unsaturated, branched and/or unbranched alcohols having a chain length of from 3 to 30 carbon atoms can also be used.

The oil phase can also advantageously be selected from the group of the branched and unbranched hydrocarbons and waxes, the silicone oils, the dialkyl ethers, the group of the saturated or unsaturated, branched or unbranched alcohols, and also of the fatty acid triglycerides, specifically the triglycerol esters of saturated and/or unsaturated, branched and/or unbranched alkanecarboxylic acids having a chain length of from 8 to 24 carbon atoms, in particular from 12 to 18 carbon atoms. The fatty acid triglycerides can advantageously be selected from the group of the synthetic, semi-synthetic and natural oils, e.g. olive oil, sunflower oil, soya oil, groundnut oil, rapeseed oil, almond oil, palm oil, coconut oil, palm kernel oil and the like. Any desired mixtures of such oil and wax components can also advantageously be used. In some cases it is also advantageous to use waxes, for example cetyl palmitate, as the only lipid component of the oil phase, the oil phase is advantageously selected from the group consisting of 2-ethylhexyl isostearate, octyldodecanol, isotridecyl isononanoate, isoeicosan, 2-ethylhexyl cocoate, C₁₂₋₁₅-alkyl benzoate, caprylic-capric acid triglyceride and dicaprylyl ether. Mixtures of C₁₂₋₁₅-alkyl benzoate and 2-ethylhexyl isostearate, mixtures of C₁₂₋₁₅-alkyl benzoate and isotridecyl isononanoate and mixtures of C₁₂₋₁₅-alkyl benzoate, 2-ethylhexyl isostearate and isotridecyl isononanoate are particularly advantageous. The hydrocarbons paraffin oil, squalane and squalene can also advantageously be used. The oil phase can advantageously also contain cyclic or linear silicone oils or consist wholly of such oils, it being preferred, however, to use an additional content of other oil-phase components in addition to the silicone oil or silicone oils. Cyclomethicone (e.g. decamethylcyclopentasiloxane) can advantageously be used as the silicone oil. However, other silicone oils can also advantageously be used, for example undecamethylcyclotrisiloxane, polydimethylsiloxane and poly(methyl-phenylsiloxane). Mixtures of cyclomethicone and isotridecyl isononanoate and also of cyclomethicone and 2-ethylhexyl isostearate are also particularly advantageous.

The aqueous phase of preparations in emulsion form that contain alkane-1,2-diols having a chain length of from C10 to C14 according to the invention can advantageously comprise: alcohols, diols or polyols having a low C number, and also ethers thereof, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, also alcohols having a low C number, e.g. ethanol, isopropanol, 1,2-propanediol, glycerol, and, in particular, one or more thickening agents, which can advantageously be selected from the group silicon dioxide, aluminium silicates, polysaccharides and derivatives thereof, e.g. hyaluronic acid, xanthan gum, hydroxypropylmethylcellulose, particularly advantageously from the group of the polyacrylates, preferably a polyacrylate from the group of the so-called carbopols, for example carbopols of types 980, 981, 1382, 2984, 5984, in each case individually or in combination.

Preparations in emulsion form that contain alkane-1,2-diols having a chain length of from C10 to C14 according to the invention advantageously comprise one or more emulsifiers. O/W emulsifiers can advantageously be selected, for example, from the group of the polyethoxylated or polypropoxylated or polyethoxylated and polypropoxylated products, e.g.:
- the fatty alcohol ethoxylates,
- the ethoxylated wool wax alcohols,
- the polyethylene glycol ethers of the general formula

   R-O-(-CH₂-CH₂-O-)ₙ-R',
- the fatty acid ethoxylates of the general formula

   R-COO-(-CH₂-CH₂-O-)ₙ-H,
- the etherified fatty acid ethoxylates of the general formula

   R-COO⁻(-CH₂-CH₂-O⁻)ₙ-R',
- the esterified fatty acid ethoxylates of the general formula

   R-COO⁻(-CH₂-CH₂-O⁻)ₙ⁻C(O)-R',
- the polyethylene glycol glycerol fatty acid esters,
- the ethoxylated sorbitan esters,
- the cholesterol ethoxylates,
- the ethoxylated triglycerides,
- the alkyl ether carboxylic acids of the general formula

   R-COO-(-CH₂-CH₂-O-)ₙ-OOH,

   and n represent a number from 5 to 30,
- the polyoxyethylenesorbitol fatty acid esters,
- the alkyl ether sulfates of the general formula

   R-O-(-CH₂-CH₂-O-)ₙ-SO₃-H,
- the fatty alcohol propoxylates of the general formula

   R-O-(-CH₂-CH(CH₃)-O-)ₙ-H,
- the polypropylene glycol ethers of the general formula

   R-O-(-CH₂-CH(CH₃)-O-)ₙ-R',
- the propoxylated wool wax alcohols,
- the etherified fatty acid propoxylates

   R-COO-(-CH₂-CH(CH₃)-O-)ₙ-R',
- the esterified fatty acid propoxylates of the general formula

   R-COO-(-CH₂-CH(CH₃)-O-)ₙ-C(O)-R',
- the fatty acid propoxylates of the general formula

   R-COO-(-CH₂-CH(CH₃)-O-)ₙ-H,
- the polypropylene glycol glycerol fatty acid esters,
- the propoxylated sorbitan esters,
- the cholesterol propoxylates,
- the propoxylated triglycerides,
- the alkyl ether carboxylic acids of the general formula

   R-O-(-CH₂-CH(CH₃)-O-)ₙ-CH₂-COOH,
- the alkyl ether sulfates or the acids on which these sulfates are based of the general formula

   R-O-(-CH₂-CH(CH₃)-O-)ₙ-SO₃-H,
- the fatty alcohol ethoxylates/propoxylates of the general formula

   R-O-Xₙ-Yₘ-H,
- the polypropylene glycol ethers of the general formula

   R-O-Xₙ-Yₘ-R',
- the etherified fatty acid propoxylates of the general formula

   R-COO-Xₙ-Yₘ-R',
- the fatty acid ethoxylates/propoxylates of the general formula

   R-COO-Xₙ-Yₘ-H.

According to the invention, the polyethoxylated or polypropoxylated or polyethoxylated and polypropoxylated O/W emulsifiers that are used are particularly advantageously selected from the group of substances having HLB values of from 11 to 18, most particularly preferably having HLB values of from 14.5 to 15.5, provided the O/W emulsifiers contain saturated radicals R and R'. If the O/W emulsifiers contain unsaturated radicals R and/or R', or if isoalkyl derivatives are present, then the preferred HLB value of such emulsifiers can also be lower or higher.

It is advantageous to select the fatty alcohol ethoxylates from the group of the ethoxylated stearyl alcohols, cetyl alcohols, cetyl stearyl alcohols (cetearyl alcohols). Particular preference is given to:
polyethylene glycol (13) stearyl ether (steareth-13), polyethylene glycol (14) stearyl ether (steareth-14), polyethylene glycol (15) stearyl ether (steareth-15), polyethylene glycol (16) stearyl ether (steareth-16), polyethylene glycol (17) stearyl ether (steareth-17), polyethylene glycol (18) stearyl ether (steareth-18), polyethylene glycol (19) stearyl ether (steareth-19), polyethylene glycol (20) stearyl ether (steareth-20), polyethylene glycol (12) isostearyl ether (isosteareth-12), polyethylene glycol (13) isostearyl ether (isosteareth-13), polyethylene glycol (14) isostearyl ether (isosteareth-14), polyethylene glycol (15) isostearyl ether (isosteareth-15), polyethylene glycol (16) isostearyl ether (isosteareth-16), polyethylene glycol (17) isostearyl ether (isosteareth-17), polyethylene glycol (18) isostearyl ether (isosteareth-18), polyethylene glycol (19) isostearyl ether (isosteareth-19), polyethylene glycol (20) isostearyl ether (isosteareth-20), polyethylene glycol (13) cetyl ether (ceteth-13), polyethylene glycol (14) cetyl ether (ceteth-14), polyethylene glycol (15) cetyl ether (ceteth-15), polyethylene glycol (16) cetyl ether (ceteth-16), polyethylene glycol (17) cetyl ether (ceteth-17), polyethylene glycol (18) cetyl ether (ceteth-18), polyethylene glycol (19) cetyl ether (ceteth-19), polyethylene glycol (20) cetyl ether (ceteth-20), polyethylene glycol (13) isocetyl ether (isoceteth-13), polyethylene glycol (14) isocetyl ether (isoceteth-14), polyethylene glycol (15) isocetyl ether (isoceteth-15), polyethylene glycol (16) isocetyl ether (isoceteth-16), polyethylene glycol (17) isocetyl ether (isoceteth-17), polyethylene glycol (18) isocetyl ether (isoceteth-18), polyethylene glycol (19) isocetyl ether (isoceteth-19), polyethylene glycol (20) isocetyl ether (isoceteth-20), polyethylene glycol (12) oleyl ether (oleth-12), polyethylene glycol (13) oleyl ether (oleth-13), polyethylene glycol (14) oleyl ether (oleth-14), polyethylene glycol (15) oleyl ether (oleth-15), polyethylene glycol (12) lauryl ether (laureth-12), polyethylene glycol (12) isolauryl ether (isolaureth-12), polyethylene glycol (13) cetylstearyl ether (ceteareth-13), polyethylene glycol (14) cetylstearyl ether (ceteareth-14), polyethylene glycol (15) cetylstearyl ether (ceteareth-15), polyethylene glycol (16) cetylstearyl ether (ceteareth-16), polyethylene glycol (17) cetylstearyl ether (ceteareth-17), polyethylene glycol (18) cetylstearyl ether (ceteareth-18), polyethylene glycol (19) cetylstearyl ether (ceteareth-19), polyethylene glycol (20) cetylstearyl ether (ceteareth-20).

It is further advantageous to select the fatty acid ethoxylates from the following group:
polyethylene glycol (20) stearate, polyethylene glycol (21) stearate, polyethylene glycol (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate, polyethylene glycol (15) oleate, polyethylene glycol (16) oleate, polyethylene glycol (17) oleate, polyethylene glycol (18) oleate, polyethylene glycol (19) oleate, polyethylene glycol (20) oleate.

Sodium laureth-11 carboxylate can advantageously be used as the ethoxylated alkyl ether carboxylic acid or salt thereof. Sodium laureth 1-4 sulfate can advantageously be used as the alkyl ether sulfate. Polyethylene glycol (30) cholesteryl ether can advantageously be used as the cholesterol derivative. Polyethylene glycol (25) soya sterol has also proved suitable.

Polyethylene glycol (60) evening primrose glycerides can advantageously be used as ethoxylated triglycerides.

It is further advantageous to select the polyethylene glycol glycerol fatty acid esters from the group polyethylene glycol (20) glyceryl laurate, polyethylene glycol (21) glyceryl laurate, polyethylene glycol (22) glyceryl laurate, polyethylene glycol (23) glyceryl laurate, polyethylene glycol (6) glyceryl caprylate/caprinate, polyethylene glycol (20) glyceryl oleate, polyethylene glycol (20) glyceryl isostearate, polyethylene glycol (18) glyceryl oleate/cocoate.

It is likewise advantageous to select the sorbitan esters from the group polyethylene glycol (20) sorbitan monolaurate, polyethylene glycol (20) sorbitan monostearate, polyethylene glycol (20) sorbitan monoisostearate, polyethylene glycol (20) sorbitan monopalmitate, polyethylene glycol (20) sorbitan monooleate.

The following can be used as advantageous W/O emulsifiers: fatty alcohols having from 8 to 30 carbon atoms, monoglycerol esters of saturated and/or unsaturated, branched and/or unbranched alkanecarboxylic acids having a chain length of from 8 to 24 carbon atoms, especially from 12 to 18 carbon atoms, diglycerol esters of saturated and/or unsaturated, branched and/or unbranched alkanecarboxylic acids having a chain length of from 8 to 24 carbon atoms, especially from 12 to 18 carbon atoms, monoglycerol ethers of saturated and/or unsaturated, branched and/or unbranched alcohols having a chain length of from 8 to 24 carbon atoms, especially from 12 to 18 carbon atoms, diglycerol ethers of saturated and/or unsaturated, branched and/or unbranched alcohols having a chain length of from 8 to 24 carbon atoms, especially from 12 to 18 carbon atoms, propylene glycol esters of saturated and/or unsaturated, branched and/or unbranched alkanecarboxylic acids having a chain length of from 8 to 24 carbon atoms, especially from 12 to 18 carbon atoms, and also sorbitan esters of saturated and/or unsaturated, branched and/or unbranched alkanecarboxylic acids having a chain length of from 8 to 24 carbon atoms, especially from 12 to 18 carbon atoms.

Particularly advantageous W/O emulsifiers are glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, saccharose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprinate, glyceryl monocaprylate.

### Preferred formulations:

### Combination with sun-protection agents:

For use, a sufficient amount of the cosmetic and/or dermatological/keratological formulations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, is applied to the skin and/or hair in the conventional manner for cosmetics and dermatics. Particular advantages are also provided by cosmetic and/or dermatological preparations which contain one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, and in addition one, two, three or more compounds that act as sun-protection agents. Such preparations advantageously contain at least one UVA filter and/or at least one UVB filter and/or at least one inorganic pigment. The preparations can be present in various forms, such as, for example, are conventionally used for this type of preparation. For example, they can preferably be a solution, an emulsion of the water-in-oil (W/O) or oil-in-water (O/W) type, or a multiple emulsion, for example of the water-in-oil-in-water (W/O/W) type, a gel, a hydrodispersion, a solid stick or alternatively an aerosol.

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can advantageously also be combined with one, two, three or more thickeners. Suitable thickeners are selected from the group consisting of homopolymers of acrylic acid having a molecular weight of from 2,000,000 to 6,000,000, preferably commercial carbopols. Further preferred thickeners are marketed under the names Carbopol 940, Carbopol EDTA 2001 or Modarez V 600 PX. Polymers of acrylic acid and acrylamide (sodium salt) having a molecular weight of from 2,000,000 to 6,000,000, such as, for example, Hostacerin PN 73 or the sclerotium gum marketed under the name Amigel. Also suitable are copolymers of acrylic acid or methacrylic acid, such as, for example, Carbopol 1342 or Permulen TRI. Further types of thickener are polyglycols, cellulose derivatives, especially hydroxyalkylcelluloses, and also alginates, carageenan and inorganic thickeners such as, for example, natural or synthetic bentonites.

### Combination with cosmetic auxiliary substances:

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can advantageously also be combined in cosmetic preparations with one, two, three or more cosmetic auxiliary substances as are conventionally used in such preparations, preferably antioxidants, perfume oils, agents for preventing foaming, colourings, pigments having a colouring action, thickening agents, surface-active substances, emulsifiers, plasticising substances, further moisturising and/or humectant substances, fats, oils, waxes or other conventional constituents of a cosmetic formulation, such as preferably alcohols, polyols, polymers, foam stabilisers, electrolytes, organic solvents or silicone derivatives. According to the invention it is possible to use any conceivable antioxidants, perfume oils, agents for preventing foaming, colourings, pigments having a colouring action, thickening agents, surface-active substances, emulsifiers, plasticising substances, moisturising and/or humectant substances, fats, oils, waxes, alcohols, polyols, polymers, foam stabilisers, electrolytes, organic solvents or silicone derivatives that are suitable or conventional for cosmetic and/or dermatological applications.

### Combination with fragrances:

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can also be used as a constituent of fragrance compositions for hair and/or scalp care products and in particular, because of their specific activity, can impart an additional anti-dandruff action, for example, to a perfumed finished product. Particularly preferred fragrance compositions comprise or consist of (a) a sensorially effective amount of a fragrance or of the sum of two, three or more fragrances, (b) a dandruff-reducing amount of one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, and (c) optionally one or more carriers and/or additives.

Because the amount of fragrance composition (perfume) in a cosmetic finished product is frequently in the region of about 1 wt.%, a fragrance composition preferably contains from 0.1 to 10 wt.% of one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, in each case based on the total weight of the fragrance composition.

It has proved particularly advantageous that the C10-C14-alkane-1,2-diols according to the invention possess only a weak inherent odour or are even completely odourless; because this property predisposes them in particular to use in a fragrance composition.

Fragrances which are advantageously suitable for combination are found, for example, in S. Arctander, Perfume and Flavor Materials, Vol. I and II, Montclair, N. J. 1969, author/publisher, or K. Bauer et al., Common Fragrance and Flavor Materials, 4th Edition, Wiley-VCH, Weinheim 2001. The following may be mentioned specifically as being preferred:
**extracts from natural raw materials** such as essential oils, concretes, absolutes, resins, resinoids, balsams, tinctures, such as, for example:
   amber tincture; amyris oil; angelica seed oil; angelica root oil; aniseed oil; valerian oil; basil oil; tree moss absolute; bay oil; artemisia oil; benzoin resin; bergamot oil; beeswax absolute; birch tar oil; bitter almond oil; summer savory oil; buchu oil; cabreuva oil; oil of cade; calamus oil; camphor oil; cananga oil; cardamom oil; cascarilla oil; oil of cassia; cassia absolute; castoreum absolute; cedar leaf oil; cedarwood oil; cistus oil; citronella oil; lemon oil; copaiva balsam; copaiva balsam oil; coriander oil; costus root oil; cumin oil; cypress oil; davana oil; dill oil; dill seed oil; eau de brouts absolute; oak moss absolute; elemi oil; tarragon oil; Eucalyptus citriodora oil; eucalyptus oil; fennel oil; spruce-needle oil; galbanum oil; galbanum resin; geranium oil; grapefruit oil; guaiacwood oil; gurjun balsam; gurjun balsam oil; helichrysum absolute; helichrysum oil; ginger oil; orris root absolute; orris root oil; jasmine absolute; calamus oil; blue camomile oil; Roman camomile oil; carrot seed oil; cascarilla oil; pine-needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linaloa oil; litseacubeba oil; oil of laurel leaves; oil of mace; marjoram oil; mandarin oil; massoia bark oil; mimosa absolute; musk seed oil; musk tincture; oil of clary sage; nutmeg oil; myrrh absolute; myrrh oil; myrtle oil; clove leaf oil; clove flower oil; Neroli oil; olibanum absolute; olibanum oil; opopanax oil; orange blossom absolute; orange oil; origanum oil; palmarosa oil; patchouli oil; perilla oil; Peru balsam oil; parsley leaf oil; parsley seed oil; oil of petitgrain; peppermint oil; pepper oil; pimento oil; pine oil; poley oil; rose absolute; rosewood oil; rose oil; rosemary oil; oil of Dalmatian sage; oil of Spanish sage; sandalwood oil; celery seed oil; spike oil; star anise oil; styrax oil; tagetes oil; fir-needle oil; tea tree oil; terpentine oil; thyme oil; tolu balsam; tonka absolute; tuberose absolute; vanilla extract; violet leaf absolute; verbena oil; vetiver oil; oil of juniper berries; grapeseed oil; vermouth oil; oil of wintergreen; ylang oil; oil of hyssop; civet absolute; cinnamon leaf oil; cinnamon bark oil as well as fractions thereof, or ingredients isolated therefrom;
   **individual fragrances** from the group of the hydrocarbons, such as, for example, 3-carene; α-pinene; β-pinene; α-terpinene; γ-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
   of the aliphatic alcohols, such as, for example, hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
   of the aliphatic aldehydes and their acetals, such as, for example, hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanaldiethylacetal; 1,1-dimethoxy-2,2,5-trimethyl-4-hexene; citronellyloxyacetaldehyde; 1-(1-methoxy-propoxy)-(E/Z)-3-hexene;
   of the aliphatic ketones and their oximes, such as, for example, 2-heptanone; 2-octanone; 3-octanone; 2-nonanone; 5-methyl-3-heptanone; 5-methyl-3-heptanoneoxime; 2,4,4,7-tetramethyl-6-octen-3-one; 6-methyl-5-hepten-2-one;
   of the aliphatic sulfur-containing compounds, such as, for example, 3-methylthio-hexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
   of the aliphatic nitriles, such as, for example, 2-nonenoic acid nitrile; 2-undecenoic acid nitrile; 2-tridecenoic acid nitrile; 3,12-tridecadienoic acid nitrile; 3,7-dimethyl-2,6-octadienoic acid nitrile; 3,7-dimethyl-6-octenoic acid nitrile;
   of the esters of aliphatic carboxylic acids, such as, for example, (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl-2-methyl pentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxyacetate; methyl 3,7-dimethyl-2,6-octadienoate; 4-methyl 2-pentyl-crotonate;
   of the acyclic terpene alcohols, such as, for example, citronellol; geraniol; nerol; linalool; lavandulol; nerolidol; farnesol; tetrahydrolinalool; tetrahydrogeraniol; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and their formates, acetates, propionates, isobutyrates, butyrates, isovalerates, pentanoates, hexanoates, crotonates, tiglinates and 3-methyl-2-butenoates;
   of the acyclic terpene aldehydes and ketones, such as, for example, geranial; neral; citral; citronellal; 7-hydroxy-3,7-dimethyloctanal; 7-methoxy-3,7-dimethyloctanal; 2,6,10-trimethyl-9-undecenal; geranylacetone; and the dimethyl- and diethyl-acetals of geranial, neral, 7-hydroxy-3,7-dimethyloctanal;
   of the cyclic terpene alcohols, such as, for example, menthol; isopulegol; alpha-terpineol; terpinenol-4; menthan-8-ol; menthan-1-ol; menthan-7-ol; borneol; isoborneol; linalool oxide; nopol; cedrol; ambrinol; vetiverol; guaiol; and their formates, acetates, propionates, isobutyrates, butyrates, isovalerates, pentanoates, hexanoates, crotonates, tiglinates and 3-methyl-2-butenoates;
   of the cyclic terpene aldehydes and ketones, such as, for example, menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalen-8(5H)-one; 2-methyl-4-(2,6,6-trimethyl-1-cyclohexen-1-yl)-2-butenal; nootcatone; dihydronootcatone; 4,6,8-megastigmatrien-3-one; alpha-sinensal; beta-sinensal; acetylated cedar wood oil (methyl cedryl ketone);
   of the cyclic alcohols, such as, for example, 4-tert.-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
   of the cycloaliphatic alcohols, such as, for example, alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
   of the cyclic and cycloaliphatic ethers, such as, for example, cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodeca-hydronaphtho[2,1-b]furan; 1,5,9-trimethyl-13-oxabicyclo[10.1.0]trideca-4,8-diene; rose oxide; 2-(2,4-dimethyl-3-cyclohexen-1-yl)-5-methyl-5-(1-methylpropyl)-1,3-dioxane;
   of the cyclic and macrocyclic ketones, such as, for example, 4-tert.-butylcyclohexanone; 2,2,5-trimethyl-5-pentylcyclopentanone; 2-heptylcyclo-pentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert.-pentylcyclohexanone; 5-cyclohexadecen-1-one; 6,7-dihydro-1,1,2,3,3-pentamethyl-4(5H)-indanone; 8-cyclohexadecen-1 -one; 9-cycloheptadecen-1-one; cyclopentadecanone; cyclohexadecanone;
   of the cycloaliphatic aldehydes, such as, for example, 2,4-dimethyl-3-cyclohexenecarbaldehyde; 2-methyl-4-(2,2,6-trimethyl-cyclohexen-1-yl)-2-butenal; 4-(4-hydroxy-4-methylpentyl)-3-cyclohexenecarbaldehyde; 4-(4-methyl-3-penten-1-yl)-3-cyclohexenecarbaldehyde;
   of the cycloaliphatic ketones, such as, for example, 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl 2,6,10-trimethyl-2,5,9-cyclododecatrienyl ketone; tert.-butyl (2,4-dimethyl-3-cyclohexen-1-yl) ketone;
   of the esters of cyclic alcohols, such as, for example, 2-tert.-butylcyclohexyl acetate; 4-tert.-butylcyclohexyl acetate; 2-tert.-pentylcyclohexyl acetate; 4-tert.-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5- or -6-indenyl acetate; 4,7-methano-3a,4,5,6,7,7a-hexahydro-5- or -6-indenyl propionate; 4,7-methano-3a,4,5,6,7,7a-hexahydro-5- or -6-indenyl isobutyrate; 4,7-methanooctahydro-5- or -6-indenyl acetate;
   of the esters of cycloaliphatic alcohols, such as, for example, 1-cyclohexylethyl crotonate;
   of the esters of cycloaliphatic carboxylic acids, such as, for example, allyl-3-cyclohexyl propionate; allylcyclohexyloxy acetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolan-2-acetate;
   of the araliphatic alcohols, such as, for example, benzyl alcohol; 1-phenylethyl alcohol; 2-phenylethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
   of the esters of araliphatic alcohols and aliphatic carboxylic acids, such as, for example, benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
   of the araliphatic ethers, such as, for example, 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
   of the aromatic and araliphatic aldehydes, such as, for example, benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethyl-propanal; 2-methyl-3-(4-isopropylphenyl)propanal; 2-methyl-3-(4-tert.-butylphenyl)propanal; 2-methyl-3-(4-isobutylphenyl)propanal; 3-(4-tert.-butylphenyl)-propanal; cinnamaldehyde; alpha-butylcinnamaldehyde; alpha-amylcinnam-aldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxy-benzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)-propanal;
   of the aromatic and araliphatic ketones, such as, for example, acetophenone; 4-methylacetophenone; 4-methoxyacetophenone; 4-tert.-butyl-2,6-dimethyl-acetophenone; 4-phenyl-2-butanone; 4-(4-hydroxyphenyl)-2-butanone; 1-(2-naphthalenyl)ethanone; 2-benzofuranylethanone; (3-methyl-2-benzofuranyl)-ethanone; benzophenone; 1,1,2,3,3,6-hexamethyl-5-indanyl methyl ketone; 6-tert.-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
   of the aromatic and araliphatic carboxylic acids and their esters, such as, for example, benzoic acid; phenylacetic acid; methyl benzoate; ethyl benzoate; hexyl benzoate; benzyl benzoate; methylphenyl acetate; ethylphenyl acetate; geranylphenyl acetate; phenylethyl-phenyl acetate; methyl cinnamate; ethyl cinnamate; benzyl cinnamate; phenylethyl cinnamate; cinnamyl cinnamate; allylphenoxy acetate; methyl salicylate; isoamyl salicylate; hexyl salicylate; cyclohexyl salicylate; cis-3-hexenyl salicylate; benzyl salicylate; phenylethyl salicylate; methyl-2,4-dihydroxy-3,6-dimethyl benzoate; ethyl-3-phenyl glycidate; ethyl-3-methyl-3-phenyl glycidate;
   of the nitrogen-containing aromatic compounds, such as, for example, 2,4,6-trinitro-1,3-dimethyl-5-tert.-butylbenzene; 3,5-dinitro-2,6-dimethyl-4-tert.-butylacetophenone; cinnamic acid nitrile; 3-methyl-5-phenyl-2-pentenoic acid nitrile; 3-methyl-5-phenylpentanoic acid nitrile; methyl anthranilate; methyl-N-methyl anthranilate; Schiff's bases of methyl anthranilate with 7-hydroxy-3,7-dimethyloctanal, 2-methyl-3-(4-tert.-butylphenyl)propanal or 2,4-dimethyl-3-cyclohexenecarbaldehyde; 6-isopropylquinoline; 6-isobutylquinoline; 6-sec.-butylquinoline; 2-(3-phenylpropyl)pyridine; indole; scatole; 2-methoxy-3-isopropylpyrazine; 2-isobutyl-3-methoxypyrazine;
   of the phenols, phenyl ethers and phenyl esters, such as, for example, estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresylphenyl acetate;
   of the heterocyclic compounds, such as, for example, 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
   of the lactones, such as, for example, 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene-1,12-dodecane dioate; ethylene-1,13-tridecane dioate; coumarin; 2,3-dihydrocoumarin; octahydrocoumarin.

Preferred carriers are ethanol, isopropanol, diethylene glycol monoethyl ether, glycerol, propylene glycol, 1,2-butylene glycol, dipropylene glycol, diethyl phthalate, triethyl citrate, isopropyl myristate and triacetin.

One or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can be incorporated without difficulty into conventional cosmetic or dermatological/keratological formulations such as, *inter alia,* pump sprays, aerosol sprays, creams, shampoos, ointments, tinctures, lotions and the like. It is also possible, and in some cases advantageous, to combine one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, with further active ingredients. Cosmetic and/or dermatological/keratological formulations containing one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, can otherwise be composed in the conventional manner and can be used to treat the skin and/or hair within the scope of a dermatological/keratological treatment or a treatment within the scope of caring cosmetics.

Preferred forms of the mixtures according to the invention and of the methods and uses according to the invention will be found in the examples which follow and the associated tables:

### Examples

The examples which follow are intended to illustrate the present invention without limiting it. Unless indicated otherwise, all amounts, parts and percentages are based on the weight and the total amount, or on the total weight of the preparations.

### Example 1

### Studies of the antimicrobial action of alkane-1,2-diols having a chain length of from C5 to C18 against Malassezia furfur (synonym: Pityrosporum ovale)

### General test conditions (MIC value measurements):

The antimicrobial action of the alkane-1,2-diols against Malassezia furfur was determined by means of the agar dilution method in accordance with DIN 58 940/ICS and DIN 58 944/ICS. Malassezia furfur strain DSM6171 was used as the test organism.

Petri dishes having a diameter of 9 cm and containing 8.7 ml of freshly prepared Mueller-Hinton agar (Merck, art. 1.05437) kept liquid at 50°C and containing 3 % Tween 80 (Merck, art. 8.2217) were used. In order to prepare the test concentrations, ethanolic stock solutions of the alkane-1,2-diol samples were diluted with 96 % ethanol until the following final test concentrations of the alkane-1,2-diols in the Mueller-Hinton agar were present: 25,000 ppm, 12,500 ppm, 6300 ppm, 3600 ppm, 1800 ppm, 1100 ppm, 900 ppm, 450 ppm, 225 ppm, 112 ppm, 56 ppm, 28 ppm, 14 ppm, 7 ppm, 4 ppm. 2 agar plates were cast per test concentration and nutrient medium.

After solidification and drying (about 1 hour at 37°C), the test plates were inoculated punctually with in each case 1 µl of the Malassezia furfur (DSM 6171 - 2.8x10⁷ KBE/ml) test organism suspensions. The inoculated plates were subsequently incubated for 72 hours at 30°C and then evaluated. The lowest active ingredient concentration at which, macroscopically, no growth is present was regarded as the MIC (minimum inhibitory concentration).

The MIC values of the alkane-1,2-diols listed in Table 1 were determined in accordance with the general test conditions.

**Table 1:**

| **Test compound** | **MIC value (ppm)** |
|---|---|
| 1,2-Pentanediol | 25,000 |
| 1,2-Hexanediol | 12,500 |
| 1,2-Octanediol | 225 |
| 1,2-Decanediol | 28 |
| 1,2-Dodecanediol | 14 |
| 1,2-Tetradecanediol | 7 |
| 1,2-Hexadecanediol | 1100 |
| 1,2-Octadecanediol | >1100 |

As the studies of the antimicrobial activity of alkane-1,2-diols show, 1,2-pentanediol (MIC value: 25,000 ppm) and 1,2-hexanediol (MIC value: 12,500 ppm) have very weak antimicrobial activity against *Malassezia furfur* (synonym: *Pityrosporum ovale).* 1,2-Hexadecanediol (MIC value: 1100 ppm) and 1,2-octadecanediol (MIC value: >1100 ppm) likewise have only extremely weak activity. The strongest antimicrobial activity against *Malassezia furfur* could be demonstrated for 1,2-decanediol, 1,2-dodecanediol and 1,2-tetradecanediol. For example, the activity of 1,2-decanediol, which has an MIC value of 28 ppm, is greater than that of 1,2-octanediol (MIC value: 225 ppm) by a factor of about 8. The activity of 1,2-dodecanediol, which has an MIC value of 14 ppm, is greater than that of 1,2-octanediol (MIC value: 225 ppm) by a factor of about 16, and the activity of 1,2-tetradecanediol, which has an MIC value of <7 ppm, is greater than that of 1,2-octanediol (MIC value: 225 ppm) by a factor of at least 32. Among the alkane-1,2-diols studied, 1,2-tetradecanediol was found to have the strongest activity against *Malassezia furfur* (synonym: *Pityrosporum ovale*); 1,2-decanediol and 1,2-dodecanediol, with MIC values markedly <100 ppm, are also to be classified in the category of anti-dandruff agents that have very good activity against *Malassezia furfur.*

### Example 2

Example formulations for preparations for the prophylaxis and/or treatment of Malassezia-induced dandruff comprise one or more, preferably one, two or three, C10-C14-alkanediols according to the invention, more preferably a compound or a mixture of two or three compounds from the group consisting of 1,2-decanediol, 1,2-dodecanediol and 1,2-tetradecanediol, as well as one, two, three, four, five, six, seven, eight, nine, ten, eleven or twelve further anti-dandruff agents selected from the group consisting of climbazole, ketoconazole, zinc pyrithione, coal tar, sulfur, selenium disulfide, octopirox (INCI: piroctone olamine), cyclopiroxolamine, undeylenic acid and the metal salts thereof, ichthyol (sulfonated shale oil), Sensiva SC-50 (ethylhexylglycerin) and/or vetikol (4-methyl-4-phenyl-2-pentanol).

| **NAME OF RAW MATERIAL (supplier)** | **INCI** | **WT.%** | | | | | | | |
|---|---|---|---|---|---|---|---|---|---|
| | | **1** | **2** | **3** | **4** | **5** | **6** | **7** | **8** |
| Allantoin (Merck) | Allantoin | - | - | - | 0.1 | - | - | - | - |
| Aloe Vera Gel Concentrate 10/1 (Symrise) | Water (Aqua), Aloe Barbadensis Leaf Juice | 1,0 | - | - | 0.5 | - | - | - | - |
| AMP-95 (Angus) | Aminomethylpropanol | - | - | - | - | - | 0.7 | - | - |
| Antil 141 Liquid (Degussa-Goldschmidt) | Propylene Glycol, PEG-55 Propylene Glycol Distearate | - | 1.0 | - | - | - | - | - | - |
| Antil 200 (Degussa-Goldschmidt) | PEG-200 Hydrogenated Glyceryl Palmitate, PEG-7 Glyceryl Cocoate | 1.5 | - | - | - | - | - | - | - |
| Bisabolol, (-) alpha (Symrise) | Bisabolol | - | - | - | - | - | - | 0.2 | |
| Carbopol ETD 2001 (Noveon) | Carbomer | - | - | - | - | 0.7 | - | - | - |
| Cetiol OE (Cognis) | Dicaprylyl Ether | - | - | 7.2 | - | - | - | - | - |
| Dracorin GMS (Symrise) | Glyceryl Stearate | - | - | - | - | - | - | - | 4.0 |
| Cetiol S (Cognis) | Diethylhexylcyclohexane | - | - | 7.0 | - | - | - | - | - |
| Cremogen AlphaPulp (Symrise) | Water (Aqua),Butylene Glycol, Malic Acid, Prunus Amygdalus Dulcis (Sweet Almond) Seed Extract, Actinidia Chinensis (Kiwi) Fruit Juice, Citrus Aurantium Dulcis (Orange) Juice, Citrus Paradisi (Grapefruit) Juice, Pyrus Malus (Apple) Juice, PEG-40 Hydrogenated Castor Oil | - | - | - | 1.0 | - | - | - | - |
| Crinipan AD (Symrise) | Climbazole | - | - | 0.3 | - | 0.5 | - | - | - |
| Cyclopiroxolamine | Cyclopiroxolamine | | | | | | | | |
| Decanediol, 1,2 | 1,2 Decanediol | 5.0 | - | - | 1.0 | - | 0.2 | 0.1 | 1.0 |
| Dehyquart A CA (Cognis) | Cetrimonium Chloride | - | 1.0 | 4.0 | - | - | - | - | - |
| Dehyton K (Cognis) | Cocoamidopropyl Betaine | 6.0 | 8.0 | - | - | - | - | - | - |
| Dihydroavenanthramid D (Symrise) | Dihydroavenanthramide D | 0.05 | - | - | - | 0.05 | - | - | 0.05 |
| Dodecanediol, 1,2 | | - | 1.0 | 0.5 | 2.0 | 0.1 | - | 0.1 | - |
| D-Panthenol 75L (DSM Nutritional) | Panthenol | - | 1.0 | 1.0 | - | - | - | - | - |
| Dracorin CE (Symrise) | Glyceryl Stearate Citrate | - | - | - | 0.3 | - | - | - | - |
| Dracorin GOC (Symrise) | Glyceryl Oleate Citrate | - | - | - | 0.5 | - | - | - | - |
| Dracorin 100 s.e. P (Symrise) | PEG-100 Stearate, Glyceryl Stearate | - | - | 1.0 | - | - | - | - | 6.0 |
| Drago-Beta-Glucan (Symrise) | Water, Butylene Glycol, Glycerin, Avena Sativa (Oat Kernel Extract) | 0.3 | 0.3 | - | - | - | - | - | - |
| Dragocare W | PEG-40 Butyloctanol Wheat | - | - | 0.5 | 1.0 | - | - | - | - |
| (Symrise) | Germ Esters, Water (Aqua), Lactic Acid, Tocopherol | | | | | | | | |
| Dragocide Liquid (Symrise) | Phenoxyethanol, Methyl,-Ethyl, Butyl, Propyl, Isobutylparaben | 0.8 | 0.8 | 0.8 | 0.8 | 0.5 | - | - | - |
| Dragoderm (Symrise) | Glycerin, Triticum Vulgare (Wheat) Gluten, Water (Aqua) | 0.3 | 1.0 | - | 2.0 | - | - | - | - |
| Dragoxat 89 (Symrise) | Ethyl Hexylisononanoate | - | - | 1.0 | - | - | - | 5.0 | - |
| Emulsiphos (Symrise) | Potassium Cetyl Phosphate, Hydrogenated Palm Glycerides | - | - | 2.0 | - | - | - | - | - |
| Ethanol 96 % | Ethanol | - | - | - | - | 30.0 | 30.0 | 0.3 | - |
| Eumulgin B1 (Cognis) | Ceteareth-12 | - | - | 3.5 | - | - | - | - | - |
| Euperlan PK 4000 (Cognis) | Glycol Distearate, Laureth-4, Cocoamidopropyl Betaine | 1.0 | 2.5 | - | - | - | - | - | - |
| Extrapone Champagne GW | Water (Aqua), Glycerin, Wine Extract, Alcohol | - | - | 2.0 | - | - | - | - | - |
| (Symrise) | | | | | | | | | |
| Extrapone Passion Flower (Symrise) | Water (Aqua), Propylene Glycol, Passiflora Incarnata Extract, Glucose | 0.5 | - | - | - | - | - | - | - |
| Farnesol (Symrise) | Farnesol | - | - | - | - | - | 0.3 | 0.1 | - |
| Frescolat ML (Symrise) | Menthyl Lactate | - | - | - | - | 0.5 | 0.3 | 0.5 | - |
| Genapol LRO liquid (Clariant) | Sodium Laureth Sulfate | 35.0 | - | - | - | - | - | - | - |
| Glycerin, 99.5 % | Glycerin | - | - | - | - | 10.0 | - | - | |
| Hair Conditioner Base (Symrise) | Cetyl Alcohol, Behentrimonium Chloride, Triticum Vulgare (Wheat) Bran Extract, Linoleic Acid | - | - | - | 3.0 | - | - | - | - |
| Hydrolite-5 (1,2 Pentanediol) (Symrise) | Pentylene Glycol | - | - | 0.5 | 0.5 | 1.0 | - | - | - |
| Ichthyol | Sodium Shale Oil Sulfonate | 0.5 | 0.5 | - | - | - | - | - | - |
| Isoadipate | Diisopropyl Adipate | - | - | - | - | 0.5 | 0.3 | - | - |
| (Symrise) | | | | | | | | | |
| Isodragol (Symrise) | Triisononanoin | - | - | - | - | - | - | 1.0 | 3.0 |
| Ketoconazole | Ketoconazole | 0.1 | - | 0.5 | - | - | - | - | 0.5 |
| PCL Liquid 100 (Symrise) | Cetearyl Octanoate | - | - | - | - | - | 0.2 | - | 2.0 |
| Luviskol K 30 Powder (BASF AG) | PVP | - | - | - | - | 3.0 | - | - | - |
| Luviskol VA 37 E (BASF AG) | PVP/VA Copolymer | - | - | - | - | - | 3.0 | - | - |
| Merquat 550 (Ondeo) | Polyquaterinium-7 | 0.5 | 1.0 | - | - | - | - | - | - |
| Mineral Oil | Mineral Oil | - | - | - | - | - | - | 88.8 | 3.0 |
| Mulsifan RT 203/80 (Z&S) | C12-15 Pareth-12 | - | - | - | - | 4.0 | - | - | - |
| Neo Heliopan AV (Symrise) | Ethylhexyl Methoxycinnamate | - | - | - | - | - | - | 0.5 | - |
| Neo Heliopan BB (Symrise) | Benzophenone-3 | 0.1 | 0.1 | - | - | - | 0.2 | - | - |
| Neo Heliopan Hydro | Phenylbenzimidazole Sulfonic | - | - | - | - | - | 0.2 | - | - |
| (Symrise) | Acid | | | | | | | | |
| Neo-PCL Water soluble N (Symrise) | Trideceth-9, PEG-5 Ethylhexanoate, Water (Aqua) | 1.0 | - | - | - | - | - | - | - |
| Neutrol TE (BASF) | Tetrahydroxypropyl Ethylenediamine | - | - | - | - | 1.0 | - | - | - |
| Octopirox | Piroctone Olamine | 0.5 | 0.5 | - | - | - | - | - | - |
| Polyquart H-81 (Cognis) | PEG-15 Cocopolyamine | - | - | 3.0 | - | - | - | - | - |
| PCL Liquid 100 (Symrise) | Cetearyl Octanoate | - | - | - | 0.5 | - | - | - | - |
| Propylene glycol | Propylene Glycol | - | - | - | - | - | - | - | 5.0 |
| Rose CL forte (Symrise) | Water (Aqua), Glycerin, PEG-40 Hydrogenated Castor Oil, Rosa Damascena Flower Oil | - | 0.5 | - | - | - | - | - | - |
| Sulfur | Sulfur | 0.1 | - | - | - | - | - | - | - |
| Selenium disulfide | Selenium Sulfide | - | - | 1.0 | - | - | - | - | - |
| Sensiva SC 50 | Ethylhexylglycerin | - | - | - | - | 0.5 | - | - | - |
| Sodium chloride | Sodium Chloride | 0.8 | 0.5 | - | - | - | - | - | - |
| Sodium hydroxide, 20 % | Sodium Hydroxide | 0.2 | 0.1 | - | 0.2 | - | - | | 8.0 |
| Solubilizer | PEG-40 Hydrogenated Castor Oil, Trideceth-9, Propylene | - | - | - | - | - | 2.0 | - | - |

| **NAME OF RAW MATERIAL (supplier)** | **INCI** | **WT.%** | | | | | | | |
|---|---|---|---|---|---|---|---|---|---|
| | | **1** | **2** | **3** | **4** | **5** | **6** | **7** | **8** |
| (Symrise) | Glycol, Water (Aqua) | | | | | | | | |
| Symdiol 68 (Symrise) | 1,2 Hexanediol, Caprylylglycol | - | - | - | - | 1.0 | - | - | - |
| Symrise Fragrance | Fragrance | 0.3 | 0.3 | 0.3 | 0.2 | 0.4 | 0.2 | 0.5 | - |
| Tetradecanediol, 1,2 | Tetradecanediol, 1,2 | - | 0.1 | 0.5 | - | - | 0.2 | 0.5 | 1.0 |
| Texapon N 70 (Cognis) | Sodium Laureth Sulfate | - | 10.0 | - | - | - | - | - | - |
| Undecylenic acid | Undecylenic Acid | 0.2 | - | 0.3 | - | - | - | - | - |
| Vetikol (Symrise) | Dimethyl Phenyl 2-Butanol | - | - | - | - | - | 1.0 | - | - |
| Water, demineralised | Water (Aqua) | Ad 100 | Ad 100 | Ad 100 | Ad 100 | Ad 100 | Ad 100 | Ad 100 | Ad 100 |
| Zinc pyrithione | Zinc Pyrithione | 1.0 | - | 0.5 | - | - | - | - | - |

| | | | | | | | | | |
|---|---|---|---|---|---|---|---|---|---|
| **1 = anti-dandruff shampoo** **2 = 2-in-1 anti-dandruff shampoo** **3 = anti-dandruff hair conditioner, leave on** **4 = anti-dandruff hair conditioner, rinse off** **5 = anti-dandruff hair setting gel** **6 = anti-dandruff hair care spray** **7 = anti-dandruff hair oil** **8 = anti-dandruff defrizzing cream** | | | | | | | | | |

## Claims

1. Use of one, two, three or more C10-C14-alkane-1,2-diols in the preparation of a composition for the prophylaxis and/or treatment of Malassezia-induced dandruff formation.

2. Use according to claim 1, **characterized in that** a compound or a mixture of two or three compounds from the group consisting of 1,2-decanediol, 1,2-dodecanediol and 1,2-tetradecanediol is selected.

3. Cosmetic and/or dermatological preparation for topical application, comprising or consisting of:
a) a dandruff-reducing amount of a total concentration of from 0.1 to 10 wt.% of one, two, three, four or more C10-C14-alkane-1,2-diols, in each case based on the total weight of the preparation, and
b) a dandruff-reducing amount of one, two, three, four or more anti-dandruff agents selected from the group consisting of benzimidazole, benzothiazole, bifonazole, butaconazole nitrate, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, sertaconazole, sulconazole nitrate, thioconazole, itraconazole, coal tar, sulfur, selenium disulfide, aluminium chloride, cyclopiroxolamine, undecylenic acid and the metal salts thereof, griseofulvin, 8-hydroxyquinoline, ciloquinol, thiobendazole, thiocarbamates, haloprogin, polyenes, hydroxypyridone, morpholine, benzylamine, allylamines, clove leaf oil, coriander oil, palmarosa oil, thyme oil, essential oil of bitter orange, cinnamaldehyde, citronellic acid, berberine, hinokitiol, birch tar oils, ichthyol, ethylhexylglycerin, 3-phenyl-1-propanol, vetikol, muguet alcohol, hexamidine diisethionate azelaic acid, isothiazolinone, and
c) optionally one, two or more auxiliary substances and/or additives.

4. Preparation according to claim 3, **characterised in that** one, two or three C10-C14-alkanediols are selected from the group consisting of 1, 2-decanediol, 1,2-dodecanediol and 1,2-tetradecanediol.

5. Preparation according to claim 3 or 4, **characterised in that** one, two or more auxiliary substances and/or additives from the group of the fragrances are selected.

6. Preparation according to any claims 3 to 5 further comprises
d) a sensorially effective amount of 1 wt.% of a fragrance or of the sum of two, three, four or more fragrances, in each case based on the total weight of the preparation.

## Patentansprüche

1. Verwendung von ein, zwei, drei oder mehr C10-C14-Alkan-1,2-diolen bei der Herstellung einer Zusammensetzung für die Prophylaxe und/oder Behandlung von Malassezia-induzierter Schuppenbildung.

2. Verwendung nach Anspruch 1, **dadurch gekennzeichnet, dass** eine Verbindung oder eine Mischung von zwei oder drei Verbindungen aus der Gruppe bestehend aus 1,2-decandiol, 1,2-dodecandiol und 1,2-tetradecandiol ausgewählt wird.

3. Kosmetische und/oder dermatologische Zubereitung für die topische Anwendung, umfassend oder bestehend aus :
a) einer Schuppen-reduzierenden Menge in einer Gesamtkonzentration von 0.1 bis 10 Gew.% von ein, zwei, drei, vier oder mehr C10-C14-Alkan-1,2-diolen, jeweils bezogen auf das Gesamtgewicht der Zubereitung, und
b) einer Schuppen-reduzierenden Menge von ein, zwei, drei, vier oder mehr Anti-Schuppen Wirkstoffen ausgewählt aus der Gruppe bestehend aus Benzimidazol, Benzothiazol, Bifonazol, Butaconazolnitrat, Clotrimazol, Croconazole, Eberconazol, Econazol, Elubiol, Fenticonazol, Fluconazol, Flutimazol, Isoconazol, Ketoconazol, Lanoconazol, Metronidazol, Miconazol, Neticonazol, Omonocolazol, Sertaconazol, Sulconazolnitrat, Thioconazol, Itraconazol, Kohlenteer, Schwefel, Selendisulfid, Aluminiumchlorid, Cyclopiroxolamin, Undecylensäure und die Metallsalze davon, Griseofulvin, 8-Hydroxychinolin, Cilochinol, Thiobendazol, Thiocarbamate, Halogenprogin, Polyene, Hydroxypyridon, Morpholin, Benzylamin, Allyla -Minen, Nelkenblattöl, Korianderöl, Palmarosaöl, Thymianöl, ätherisches Öl von Bitterorange, Zimtaldehyd, Citronellsäure, Berberin, Hinokitiol, Birken-Teeröle, Ichthyol, Ethylhexylglycerin, 3-phenyl-1-propanol, Vetikol, Muguetalkohl, Hexamidindiisethionat, Azelainsäure, Isothiazolinon
und
c) optional ein, zwei oder mehr Hilfsstoffe und/oder Zusatzstoffe.

4. Zubereitung nach Anspruch 3, **dadurch gekennzeichnet, dass** ein, zwei oder drei C10-C14-Alkandiole ausgewählt werden aus der Gruppe bestehend aus 1,2-decandiol, 1,2-dodecandiol und 1,2-tetradecandiol.

5. Zubereitung nach Anspruch 3 oder 4, **dadurch gekennzeichnet, dass** ein, zwei oder mehr Hilfsstoffe und/oder Zusatzstoffe aus der Gruppe der Duftstoffe ausgewählt werden.

6. Zubereitung nach einem der Ansprüche 3 bis 5, weiterhin umfassend
d) eine sensorisch effektive Menge von 1 Gew.% eines Duftstoffs oder einer Summe aus zwei, drei, vier oder mehr Dufstoffen, jeweils bezogen auf das Gesamtgewicht der Zubereitung.

## Revendications

1. Utilisation d'un, deux, trois, ou plus, C10-C14-alcane-1,2-diols dans la préparation d'une composition pour la prophylaxie et/ou le traitement de la formation de pellicules induite par Malassezia.

2. Utilisation selon la revendication 1, **caractérisée en ce qu'**un composé ou un mélange de deux ou trois composés choisis dans le groupe constitué par le 1,2-décanediol, le 1,2-dodécanediol et le 1,2-tétra-décanediol, est sélectionné.

3. Préparation cosmétique et/ou dermatologique pour une application topique, comprenant ou étant constituée :
a) d'une quantité de réduction des pellicules selon une concentration totale allant de 0,1 à 10% en poids d'un, deux, trois, quatre, ou plus, C10-C14-alcane-1,2-diols, dans chaque cas sur la base du poids total de la préparation, et
b) d'une quantité de réduction des pellicules d'un, deux, trois, quatre, ou plus, agents antipelliculaires choisis dans le groupe constitué par le benzimidazole, le benzothiazole, le bifonazole, le nitrate de butaconazole, le clotrimazole, le croconazole, l'éberconazole, l'éconazole, l'élubiol, le fenticonazole, le fluconazole, le flutrimazole, l'isoconazole, le kétoconazole, le lanoconazole, le métronidazole, le miconazole, le néticonazole, l'omoconazole, le sertaconazole, le nitrate de sulconazole, le thioconazole, l'itraconazole, le goudron de houille, le soufre, le disulfure de sélénium, le chlorure d'aluminium, la ciclopirox olamine, l'acide undécylénique et les sels métalliques de celui-ci, la griséofulvine, la 8-hydroxyquinoléine, le clioquinol, le thiobendazole, les thiocarbamates, l'haloprogine, les polyènes, l'hydroxypyridone, la morpholine, la benzylamine, les allylamines, l'huile de feuilles de giroflier, l'huile de coriandre, l'huile de Palmarosa, l'huile de thym, l'huile essentielle d'orange amère, le cinnamaldéhyde, l'acide citronellique, la berbérine, l'hinokitiol, les huiles de goudron de bouleau, l'ichthyol, l'éthylhexylglycérol, le 3-phényl-1-propanol, le vétikol, l'alcool de muguet, le diiséthionate d'hexamidine, l'acide azélaïque, l'isothiazolinone, et
c) éventuellement, d'un(e), deux, ou plus, substances auxiliaires et/ou additifs.

4. Préparation selon la revendication 3, **caractérisée en ce qu'**un, deux, trois C10-C14-alcanediols sont choisis dans le groupe constitué par le 1,2-décanediol, le 1,2-dodécanediol et le 1,2-tétradécanediol.

5. Préparation selon la revendication 3 ou 4, **caractérisée en ce qu'**un, deux, ou plus, substances auxiliaires et/ou additifs choisis dans le groupe constitué par les parfums, sont sélectionnés.

6. Préparation selon l'une quelconque des revendications 3 à 5, comprenant en outre
d) une quantité efficace sur le plan sensoriel de 1% en poids d'un parfum, ou de la somme de deux, trois, quatre, ou plus, parfums, dans chaque cas sur la base du poids total de la préparation.
